# FASCICULE DE BREVET EUROPEEN

(11) **EP 3 772 043 B1**
(45) Date de publication et mention de la délivrance du brevet: **03.06.2026**
(21) Numéro de dépôt: 20185399.1
(22) Date de dépôt: 13.07.2020
(51) Int. Cl.: G06T 19/00, G06T 19/20

(54) **SIMULATION AMELIOREE DU DEPLOIEMENT D'UNE ENDOPROTHESE EN TEMPS REEL**
VERBESSERTE SIMULATION DES EINSATZES EINER ENDOPROTHESE IN ECHTZEIT
IMPROVED SIMULATION OF THE REAL TIME DEPLOYMENT OF AN ENDOPROSTHESIS

(30) Priorité: 29.07.2019 FR 1908569
(43) Date de publication de la demande: 03.02.2021
(73) Titulaire: THALES, 92190 Meudon (FR); Association Pour La Recherche Et Le Développement De Méthodes Et Processus Industriels "Armines", 75272 Paris Cedex 06 (FR); Université Jean Monnet Saint-Étienne, 42023 Saint-Étienne Cedex 2 (FR); Centre Hospitalier Universitaire Saint-Etienne, 42055 Saint Etienne Cedex 2 (FR)
(72) Inventeur: PIONTECK, Aymeric, 38430 MOIRANS (FR); PIERRAT, Baptiste, 42330 AVEIZIEUX (FR); GORGES, Sébastien, 38430 MOIRANS (FR); ALBERTINI, Jean-Noël, 42290 SORBIER (FR); AVRIL, Stéphane, 42100 SAINT-ETIENNE (FR)
(74) Mandataire: Atout PI Laplace

(56) Documents cités:
- US-A1- 2005 010 105
- P. HAIGRON ET AL: "Angiovision: Aortic stent-graft placement by augmented angionavigation", IRBM, vol. 34, no. 2, 1 April 2013 (2013-04-01), AMSTERDAM, NL, pages 167 - 175, XP055700581, ISSN: 1959-0318, DOI: 10.1016/j.irbm.2013.01.016
- DAVID PERRIN ET AL: "Patient-specific numerical simulation of stent-graft deployment: Validation on three clinical cases", JOURNAL OF BIOMECHANICS, vol. 48, no. 10, 1 July 2015 (2015-07-01), US, pages 1868 - 1875, XP055696473, ISSN: 0021-9290, DOI: 10.1016/j.jbiomech.2015.04.031
- AURICCHIO F ET AL: "Patient-specific aortic endografting simulation: From diagnosis to prediction", COMPUTERS IN BIOLOGY AND MEDICINE, NEW YORK, NY, US, vol. 43, no. 4, 8 February 2013 (2013-02-08), pages 386 - 394, XP028987581, ISSN: 0010-4825, DOI: 10.1016/J.COMPBIOMED.2013.01.006

## Description

### Domaine de l'invention

La présente invention concerne le domaine des systèmes médicaux à rayon X et plus particulièrement les systèmes de radiologie utilisés pour la chirurgie endovasculaire des anévrismes de l'aorte abdominale.

### Etat de l'art précédent

Le traitement des anévrismes des aortes abdominales peut se faire par chirurgie ouverte classique ou par chirurgie endovasculaire. Durant une intervention endovasculaire, le chirurgien n'a pas un accès direct au site de l'opération. Au contraire, dans une opération endovasculaire, il incise l'aorte fémorale au niveau de l'aine, insère un câble métallique très souple. Il peut ensuite remonter en poussant ce câble ses outils, y compris l'endoprothèse jusqu'au niveau de l'anévrisme, dans l'aorte abdominale. Lorsqu'il estime que l'endoprothèse est située au bon endroit, il peut décider de larguer l'endoprothèse, qui se déploie automatiquement.

Le bon positionnement de l'endoprothèse est donc crucial. En effet, cette prothèse doit être correctement placée, et ne doit pas boucher des artères partant de l'artère principale. Dans les cas les plus complexes, des endoprothèses dites fenêtrées comprennent des ouvertures qui doivent êtres précisément positionnées en face des ostia des artères secondaires. Par exemple, ces ouvertures doivent être positionnées face aux jonctions entre l'aorte et les artères rénales qui en partent. Il est alors essentiel de positionner correctement l'endoprothèse, pour ne pas obstruer ces jonctions et boucher les artères rénales.

Afin de guider ces gestes, le chirurgien utilise un système de radiologie interventionnel mobile pour positionner précisément l'endoprothèse qui va rediriger le flux sanguin. Ces systèmes aussi appelés « mobile C-arm » (ou amplificateur de bloc) permettent au chirurgien d'acquérir des images à rayon X durant l'intervention et de contrôler en temps réel le positionnement des outils (cathéter, prothèse ...) de manière minimalement invasive. La plupart de ces systèmes permettent d'obtenir des images en deux dimensions avec un flux d'image vidéo à 30 images par seconde. Le praticien utilise ensuite ces images pour effectuer une reconstruction mentale de l'outil et de la géométrie de l'artère afin de valider son positionnement en temps réel. Cette imagerie par rayons X requiert une injection de produit de contraste. Ce produit de contraste est indispensable pour rendre l'aorte visible aux rayons X, mais présente le désavantage d'être toxique. Le nombre d'injections et de prises de vues possible est donc limité.

L'étape de placement d'une endoprothèse, très complexe dans certains cas, nécessite de multiplier les prises de vues, et par conséquent l'irradiation du patient ainsi que le volume de produit de contraste injecté. La probabilité de complications post-opératoires est également plus élevée. A court terme, la perte de sang et une mauvaise irrigation des zones obstruées par les outils chirurgicaux en sont la cause. A moyens et longs termes, un positionnement imprécis de l'endoprothèse entraine des risques de fuites et de thromboses. Le déploiement d'endoprothèse au sein de l'aorte s'effectue en 3D. Une unique vue 2D peut donc s'avérer insuffisante pour le chirurgien pour bien appréhender le déploiement des endoprothèses. Pouvoir disposer d'une visualisation en trois dimensions du site de l'opération serait donc un atout important pour le chirurgien, lui permettant de positionner ses outils rapidement et précisément, tout en réduisant le nombre d'angiographies.

Il existe actuellement plusieurs méthodes permettant cette visualisation en 3D. Cependant, celles-ci restent basées sur la prise de vue de multiples images 2D de différents angles de vue, en vue d'une reconstruction 3D. Elles impliquent donc de multiples injections de produit de contraste, ainsi qu'un allongement de la durée d'opération pour multiplier les prises de vues selon différents angles. L'article "Angiovision: Aortic stent-graft placement by augmented angionavigation" par P. Haigron et al et publié par IRBM ELSEVIER AMSTERDAM, NL en Avril 2013 décrit un système de guidage per-opératoire où un modèle 3D pré-opératoire de l'aorte est recalé et déformé par simulation par éléments finis en fonction d'un guide-fil rigide, puis utilisé pour positionner un modèle 3D d'endoprothèse et en afficher le déploiement géométrique.

Il y a donc besoin d'un outil d'assistance au geste chirurgical, permettant au chirurgien de visualiser ses outils et leur configuration dans l'aorte du patient, quasiment en temps réel et en 3D.

### Résumé de l'invention

L'invention est définie par les revendications indépendantes. A cet effet, l'invention a pour objet une méthode comprenant : la capture d'une image 2D d'une structure vasculaire par rayon X ; l'obtention d'un modèle 3D de la structure vasculaire ; l'obtention d'un modèle d'une endoprothèse dans la structure vasculaire comprenant une pluralité de stents ; la détermination, à partir de l'image 2D et pour chaque stent, d'au moins une position, au moins une orientation, et au moins une valeur de déploiement ; la simulation du déploiement dans le modèle 3D de la structure vasculaire, pour chaque stent, d'un modèle du stent représentant la structure du stent, ledit modèle du stent étant initialisé à partir du modèle de l'endoprothèse ; l'affichage des modèles de stents déployés.

Avantageusement, l'image 2D définit un référentiel 3D comprenant un axe vertical et un axe horizontal et un axe de profondeur de la prise de vue ; dans le modèle de l'endoprothèse, chaque stent est défini par : au moins une position d'au moins un point caractéristique et une orientation définies par au moins 6 degrés de liberté dont : la position 3D d'un premier point caractéristique du stent dans le référentiel ; un angle de rotation propre ; l'au moins une valeur de déploiement du stent, définie autour de son axe central ; le modèle du stent est formé d'une pluralité d'éléments poutres liés entre eux ; ladite méthode comprenant : la détermination, à partir de l'image 2D et pour chaque stent, d'au moins une position, au moins une orientation, et au moins une valeur de déploiement ; de l'au moins un point caractéristique selon les axe vertical et horizontal et l'au moins une valeur de déploiement dudit stent ; la détermination, pour au moins un stent, de son angle de rotation propre.

Avantageusement, la position et l'orientation de chaque stent sont définies par les positions 3D de trois points caractéristiques correspondant respectivement au centre, à l'extrémité supérieure et à l'extrémité inférieure du stent le long de son axe central, et la rotation propre du stent autour de son axe central.

Avantageusement, la détermination, pour au moins un stent, de son angle de rotation propre comprend la détermination de l'angle de rotation propre pour lequel au moins une projection de position 3D d'au moins un marqueur radio-opaque sur le stent correspond le plus à au moins une image de l'au moins marqueur sur l'image 2D.

Avantageusement, l'angle de rotation propre est obtenu par l'exécution d'une boucle de minimisation de la distance entre l'au moins une projection et l'au moins une image de l'au moins un marqueur sur l'image 2D, en fonction de l'angle de rotation propre.

Avantageusement, la détermination, pour au moins un stent, de son angle de rotation propre comprend la modélisation du stent sous la forme d'un élément poutre d'un modèle éléments finis, entre un point caractéristique représentant l'extrémité supérieure de l'axe central du stent et un point caractéristique représentant l'extrémité inférieure de l'axe central du stent, dans lequel les points caractéristiques sont libres de déplacements selon l'axe de profondeur.

Avantageusement, l'endoprothèse est représentée sous la forme d'un modèle éléments finis dans lequel : chaque stent est modélisé par un élément poutre, les stents successifs étant liés entre eux par au moins un élément poutre ; les positions des points caractéristiques des stents dont le diamètre maximal de déploiement est supérieur ou égal au diamètre de la structure vasculaire à la position de déploiement sont fixés selon les axes vertical, horizontal et de profondeur ; les positions des points caractéristiques des stents dont le diamètre maximal de déploiement est inférieur au diamètre de la structure vasculaire à la position de déploiement sont fixés selon les axes vertical, horizontal et libres en déplacement selon l'axe de profondeur ; les positions des points caractéristiques des stents dont le diamètre maximal de déploiement est inférieur au diamètre de la structure vasculaire à la position de déploiement selon l'axe de profondeur sont déterminés par l'équilibre mécanique du modèle éléments finis.

Avantageusement, l'affichage des modèles de stents déployés comprend l'affichage en superposition de la projection des modèles des stents sur l'image 2D de la structure vasculaire.

Avantageusement, l'affichage des modèles de stents déployés comprend l'affichage en 3D des modèles de stents déployés et du modèle 3D de la structure vasculaire.

Avantageusement, le modèle 3D de la structure vasculaire est un modèle éléments finis représentant la ligne centrale de la structure vasculaire par des éléments poutres.

L'invention décrit également un produit programme d'ordinateur comprenant des éléments de code informatique configurés pour exécuter une méthode selon l'un des modes de réalisation de l'invention.

L'invention décrit également un dispositif comprenant : au moins un port d'entrée configuré pour recevoir d'une image 2D de la structure vasculaire capturée par rayon X ; au moins une unité de calcul configurée pour exécuter une méthode selon l'un des modes de réalisation de l'invention.

La méthode, dans son ensemble, permet un calcul à la fois fiable et rapide du déploiement d'une endoprothèse dans l'aorte. Par exemple, la méthode peut s'exécuter en une trentaine de secondes sur des moyens de calcul conventionnels, ce qui permet une modélisation du déploiement de l'endoprothèse quasiment en temps réel.

La méthode de l'invention permet au chirurgien de disposer en temps réel d'une visualisation du déploiement de l'endoprothèse à partir de sa position actuelle. Ceci permet ainsi de considérablement améliorer la précision du traitement effectué par le chirurgien.

La méthode de l'invention requiert une unique prise de vue pour simuler le déploiement d'une endoprothèse, ce qui évite d'utiliser des injections multiples de produit de contraste.

La méthode est applicable aussi bien aux prothèses déployées qu'aux prothèse non ou partiellement déployées.

D'autres caractéristiques, détails et avantages de l'invention ressortiront à la lecture de la description faite en référence aux dessins annexés donnés à titre d'exemple et qui représentent, respectivement :
La figure 1 un système d'imagerie médicale dans lequel l'invention peut être implémentée ;
La figure 2a une image péri-opératoire en 2D montrant une endoprothèse dans l'aorte, avec injection de produit de contraste ;
La figure 2b une image péri-opératoire en 2D montrant une endoprothèse dans l'aorte, sans injection de produit de contraste ;
La figure 2c une endoprothèse déployée dans une maquette d'aorte selon un ensemble de modes de mise en œuvre de l'invention ;
La figure 3 une méthode de simulation du déploiement d'une endoprothèse selon un ensemble de modes de mise en œuvre de l'invention ;
La figure 4 une modélisation d'un stent au sein d'un modèle d'endoprothèse selon un ensemble de modes de mise en œuvre de l'invention ;
La figure 5 le déploiement d'un stent dans le cadre d'un modèle de l'endoprothèse ;
La figure 6 la détermination, à partir de l'image 2D, des positions verticales et horizontales des points caractéristiques des stents, ainsi que du déploiement de ceux-ci ;
La figure 7a un premier exemple de modélisation du déploiement d'un stent selon un ensemble de modes de mise en œuvre de l'invention ;
La figure 7b un deuxième exemple de modélisation du déploiement d'un stent selon un ensemble de modes de mise en œuvre de l'invention ;
La figure 8 une superposition de la simulation de déploiement d'un stent sur une image péri-opératoire selon un ensemble de modes de mise en œuvre de l'invention.

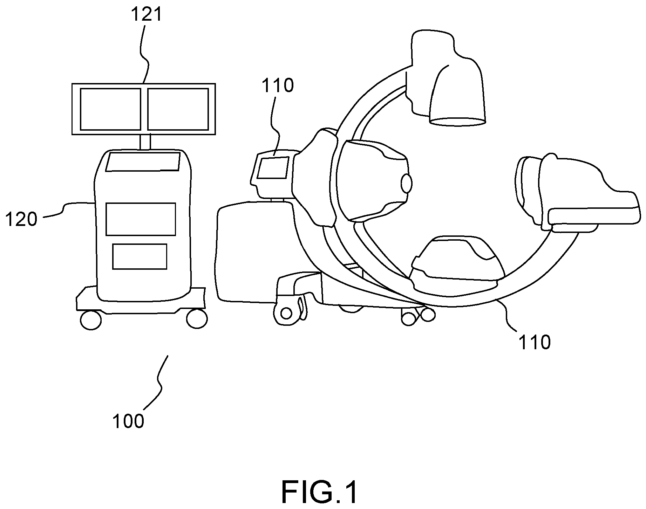

La figure 1 représente un système d'imagerie médicale dans lequel l'invention peut être implémentée.

Le système 100 est un système de radiologie interventionnel mobile.

Il comprend un élément dit C-arm 110, permettant de prendre des images en rayon X du corps d'un patient. Le C-arm est capable de tourner autour de différents axes, pour capturer des images d'un patient immobile selon différents angles. Le C-arm comprend une interface de commande permettant à l'équipe médicale de commander l'orientation et la prise de vue.

Le C-arm 100 est connecté à un dispositif de calcul, par exemple un ordinateur 120 pouvant générer l'affichage de l'image en rayons X prise par le C-arm 110 sur des moyens d'affichage, par exemple des écrans 121. Le dispositif de calcul 120 comprend à cet effet un port d'entrée pour recevoir les images péri-opératoires. Le dispositif de calcul 120 comprend également au moins un une unité de calcul (par exemple un processeur), permettant de traiter les images prises par le C-arm. Par exemple, le processeur peut effectuer une pré-analyse de l'image, superposer sur l'image des informations pouvant intéresser l'équipe médicale (par exemple l'heure de prise de vue, la température du patient, sa pression sanguine, etc.).

La séparation du système en deux dispositifs, respectivement le C-arm 110 pour prendre les images, et le dispositif de calcul 120 pour les traiter et les afficher, permet une grande flexibilité dans l'utilisation du C-arm 110 qui peut rester mobile, même si la position des écrans d'affichage 121 reste fixe.

Dans un ensemble de modes de réalisation de l'invention, ce dispositif 120 permet également de générer une visualisation du déploiement d'une endoprothèse, et de générer son affichage en 2D ou 3D, comme il sera expliqué ci-après.

Le système 100 est donné à titre d'exemple uniquement d'un système dans lequel l'invention pourrait être implémentée. L'invention pourrait être implémentée dans de nombreux autres systèmes médicaux. Par exemple, selon différents modes de prise de vue, le traitement et l'affichage des images peuvent être effectués dans un dispositif unique. Il est également possible d'effectuer la prise de vue à un endroit, et d'envoyer l'image pour son traitement et son affichage à un autre emplacement, par exemple sur un serveur distant.

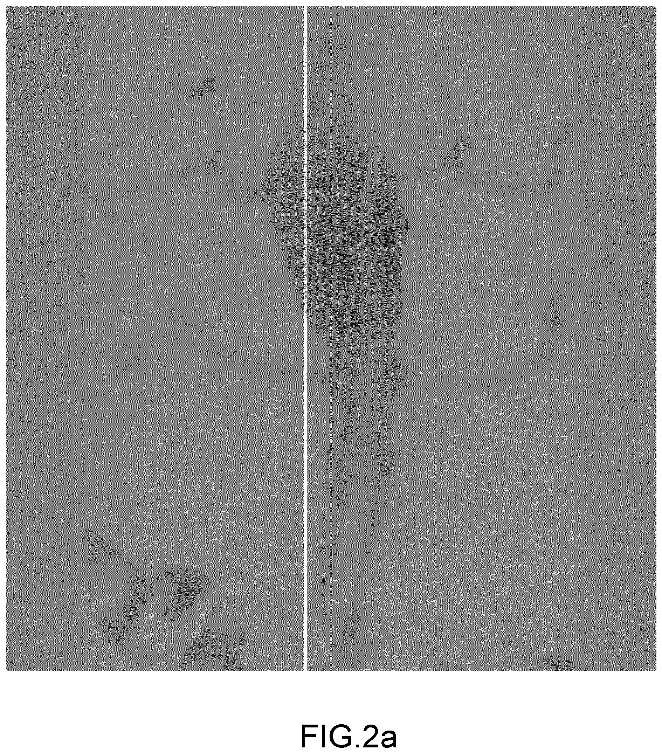

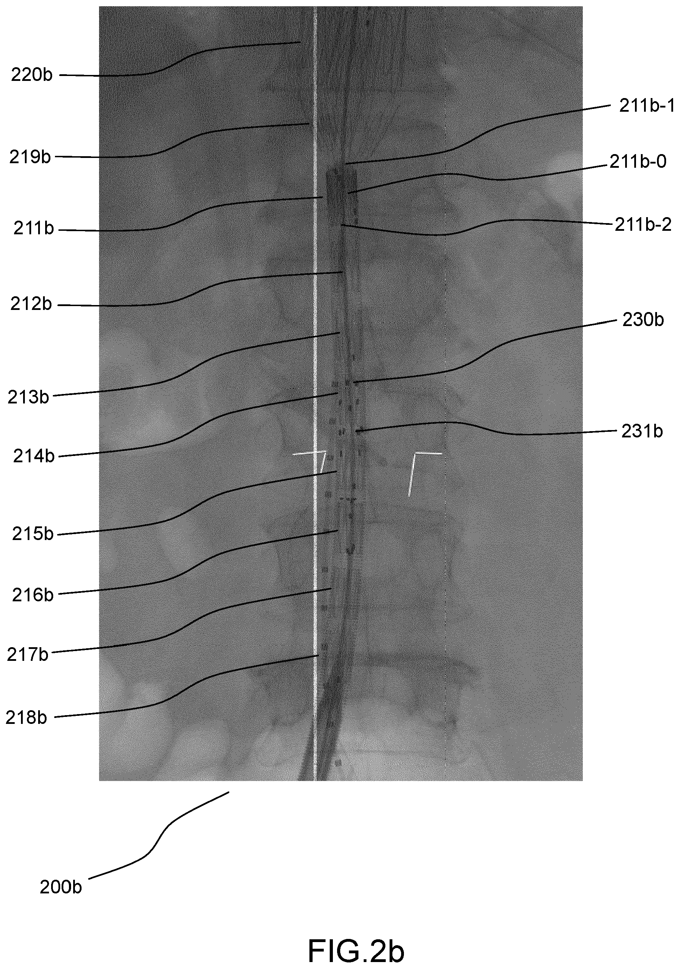

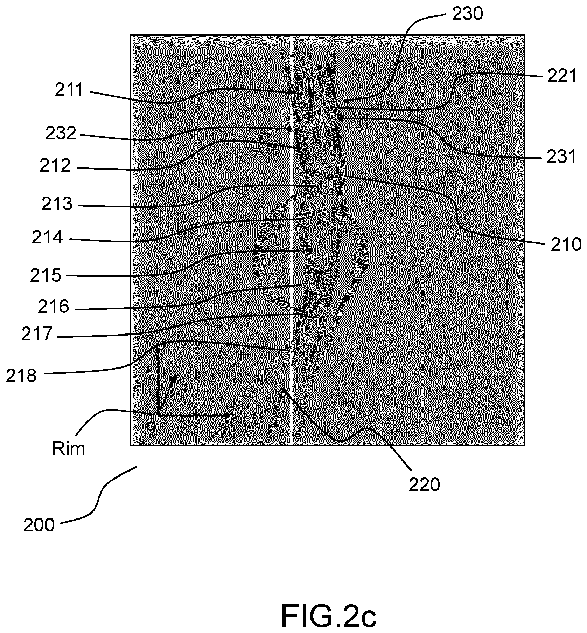

Les figures 2a, 2b et 2c représentent respectivement une image péri-opératoire en 2D montrant une endoprothèse dans l'aorte, avec et sans injection de produit de contraste, et une endoprothèse déployée dans une maquette d'aorte selon un ensemble de modes de mise en œuvre de l'invention.

La figure 2a représente une image péri-opératoire prise par rayon X, par exemple par un dispositif tel que le dispositif 100, de l'aorte prise avec produit de contraste. Cette image permet de visualiser les limites de l'aorte. En particulier, elle permet de savoir, en chaque point, que est le diamètre de l'aorte et de visualiser d'où partent les veines adjacentes.

La figure 2b représente une image péri-opératoire 200b prise par rayon X, par exemple par un dispositif tel que le dispositif 100, de l'aorte prise sans produit de contraste et avant déploiement des stents. Sans produit de contraste, on ne distingue plus les limites de l'aorte, mais les stents 211b, 212b, 231b, 214b, 215b, 216b, 217b et 218b, 219b, 220b de l'endoprothèse apparaissent. Les stents 211b à 218b ne sont pas déployés, le stent 219b est partiellement déployé, et le stent 220b est complètement déployé. Les marqueurs radio-opaques, tels que les marqueurs 230b et 231b apparaissent également. L'imagerie péri-opératoire permet également de repérer certains points caractéristiques critiques, qui peuvent par exemple être visualisés par un marqueur tel que les marqueurs 230b et 231b. Ces marqueurs peuvent être positionnés à des points particulièrement importants sur l'endoprothèse, afin de permettre au chirurgien de les repérer sur l'image péri-opératoire.

Lors de l'introduction de l'endoprothèse, le chirurgien peut effectuer une première prise de vue avec injection de produit de contraste afin de visualiser les limites de l'aorte, puis une ou plusieurs prises de vue sans produit de contraste pour visualiser la position des stents et des marqueurs radio-opaques. Ces deux vues peuvent être affichées en superposition, afin de visualiser la position des stents au sein de l'aorte.

A ce stade, l'endoprothèse peut être non déployée, partiellement déployée ou totalement déployée. Cependant, une prise de vue telle que celle de la figure 2b permet de déterminer les positions des stents, comme il sera expliqué plus bas.

Les images péri-opératoires 2D de ce type permettent donc au chirurgien d'avoir une vue générale de la position et du déploiement d'endoprothèses dans une artère. Cependant, une telle vue en 2D peut souvent s'avérer insuffisante pour appréhender un déploiement qui par nature s'opère en 3D.

La figure 2c représente une image d'une endoprothèse déployée dans une maquette. Cette image est semblable à ce que voit un chirurgien par superposition d'une image péri-opératoire avec produit de contraste, et d'une image péri-opératoire sans produit de contraste, c'est-à-dire qu'il visualise à la fois l'endoprothèse et l'espace dans lequel elle se déploie. Dans l'exemple de la figure 2c, l'endoprothèse est complètement déployée. Cette image représente une endoprothèse déployée dans une maquette de l'artère aorte 220. L'endoprothèse est formée de plusieurs stents 211, 212, 213, 214, 215, 216, 217, 218. L'image péri-opératoire permet donc de visualiser, en 2D, la position et le déploiement des stents au sein d'une artère.

Comme il sera expliqué ci-dessous, l'invention permet, à partir d'une image 2D, d'obtenir une simulation et une visualisation en 3D du déploiement d'une endoprothèse. Dans la suite de la description, le déploiement 3D sera exprimé selon un référentiel Rᵢₘ, basé sur 3 axes x, y et z, correspondant respectivement à des axes vertical, horizontal et de profondeur. Le plan formé par les axes vertical x et horizontal y est appelé plan de projection ou plan de l'image 2D. Cependant, ce référentiel est donné à titre d'exemple uniquement, et l'homme de l'art pourra, à la lecture de la présente divulgation, choisir tout référentiel 3D correspondant à ses besoins.

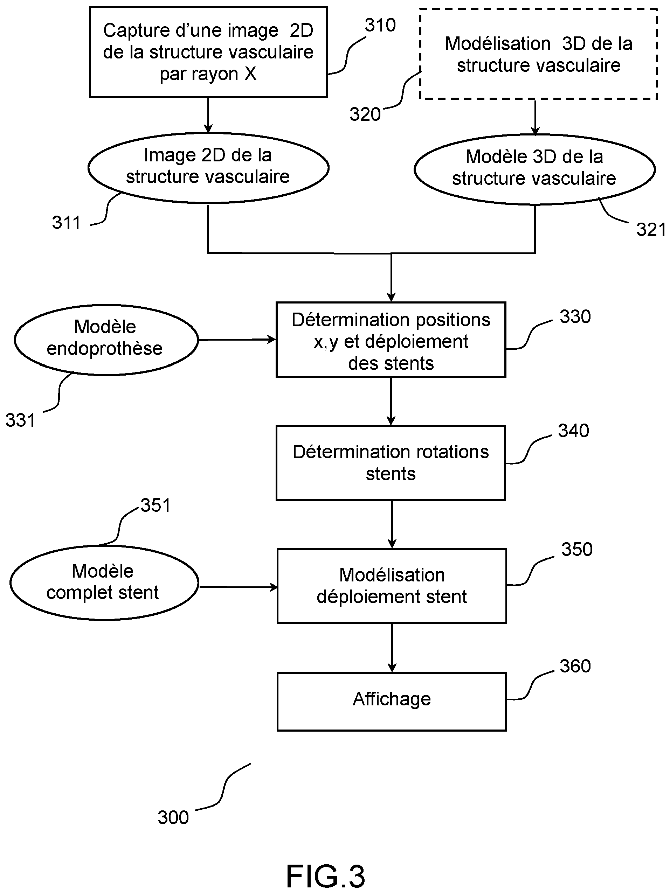

La figure 3 représente une méthode de simulation du déploiement d'une endoprothèse selon un ensemble de modes de mise en œuvre de l'invention.

La méthode 300 a pour objectif de simuler le déploiement d'une endoprothèse, et de permettre à un chirurgien de visualiser le déploiement d'une endoprothèse dans une artère, le cas échéant en 3D.

La méthode 300 comprend une étape 310 de capture d'une image 2D 311 de d'une structure vasculaire par rayon X. Cette image 2D est une image péri-opératoire, et peut par exemple être prise par un dispositif de type C-arm tel que le dispositif 100. Les images des figures 2a et 2b représentent deux exemples d'une telle image. Comme expliqué ci-dessus, la capture d'une telle image par rayon X peut comprendre une injection de produit de contraste dans la structure vasculaire. Il convient de noter que, dans la suite de la description, la méthode sera illustrée par des exemples relatifs à la simulation d'une endoprothèse dans l'aorte. Cependant, ces exemples sont donnés à titre d'exemples non limitatifs, et la méthode peut être appliquée à la simulation du déploiement d'endoprothèse dans toute structure vasculaire. Il n'existe pas de différence fondamentale entre le déploiement d'une endoprothèse dans l'aorte et dans une autre structure vasculaire, si bien que la méthode peut être directement appliquée à d'autres structures vasculaires. La méthode est également applicable à la simulation de déploiement de l'endoprothèse dans des maquettes chirurgicales.

La capture de l'image 2D définit un référentiel 3D qui sera utilisé dans la suite de la méthode. Ce référentiel 3D comprend un axe vertical, un axe horizontal et un axe de profondeur de la prise de vue. Dans l'exemple de la figure 2, ces axes sont notés respectivement x, y et z. Cependant, cette nomenclature est fournie à titre d'exemple uniquement, et l'homme de l'art pourra sans peine sélectionner les dénominations et orientations des axes correspondant à ces besoins.

La méthode 300 comprend également l'obtention d'un modèle 3D 321 de l'aorte. Ce modèle 3D 321 permet de définir les contours et le volume de l'aorte en 3D. L'aorte peut par exemple être définie par un ensemble de polygones en 3D. Les limites de l'aorte peuvent ainsi être exprimées dans le référentiel défini par l'imagerie 2D.

Selon différents modes de réalisation de l'invention, le modèle 3D peut être obtenu de différentes manières. Par exemple, il peut avoir été créé préalablement, pour le patient par imagerie 3D, obtenue préalablement puis recalée en temps réel au cours de l'opération, ou peut être créé à partir des images péri-opératoires en 2D.

Dans un ensemble de modes de réalisation de l'invention, le modèle 3D de l'aorte 321 est un modèle éléments finis, comprenant une représentation de la ligne centrale de l'aorte, et de sa surface.

Dans un ensemble de modes de mise en œuvre de l'invention, le modèle 3D de l'aorte est obtenu à partir d'une image péri-opératoire en 2D, par exemple une image obtenue à partir d'un C-arm, par une méthode de recalage non-rigide d'un modèle préalable de l'aorte.

Comme indiqué ci-dessus, la méthode de recalage non-rigide de l'aorte est basée sur un modèle aux éléments finis de la ligne centrale de l'artère. Les informations péri-opératoires contenues dans le plan de projection de l'image péri-opératoire 2D servent de conditions aux limites au modèle éléments-finis. Les déformations hors du plan sont alors calculées par le modèle mécanique.

Dans un ensemble de modes de réalisation de l'invention, la méthode de recalage présente les étapes suivantes :
- la ligne centrale de l'artère est extraite en utilisant la méthode du diagramme de Voronoï, introduite par exemple par Antiga, L. (2002). Patient-specific modeling of geometry and blood flow in large arteries. Politecnico di Milano ;
- la ligne centrale est implémentée dans un modèle éléments finis, basé sur des éléments poutres. Ceci peut être fait de différentes manières. Duriez, C. (2013). Real-time haptic simulation of medical procedures involving deformations and device-tissue interactions (Doctoral dissertation, Université des Sciences et Technologie de Lille-Lille I) fournit un exemple d'implémentation de ligne centrale de l'aorte via un modèle d'éléments finis ;
- un premier recalage non-rigide 2D/2D est effectué entre les images péri opératoires et la projection de la ligne centrale extraite à l'étape précédente. Ce recalage non-rigide est composé d'un recalage rigide puis d'une interpolation ;
- les informations 2D concernant la position de l'aorte ou des outils pendant la chirurgie sont ensuite implémentées dans le modèle éléments finis. La matrice de projection des images péri opératoires est supposée connue. Grâce à cela, il est possible de calculer les lignes de rétroprojections de chacun des points caractéristiques identifiés sur les images 2D. Puis, ces informations sont implémentées dans le modèle mécanique sous formes de conditions aux limites, les points du modèle 3D étant contraints de se déplacer le long des lignes de rétroprojection. Ainsi, le modèle mécanique calcule les déformations qui ne sont pas contenues dans le plan de projection de l'image péri-opératoire 2D pour atteindre un état d'équilibre ;
- le volume de l'aorte est alors recréé autour de la ligne centrale mise-à-jour.

Dans un ensemble de modes de réalisation de l'invention, le modèle 3D de l'aorte est superposé aux images péri-opératoires.

La méthode 300 comprend également l'obtention d'un modèle 331 de l'endoprothèse. Dans ce modèle, chaque stent est défini par au moins une position et une orientation du stent définies par au moins 6 degrés de liberté, et un déploiement du stent.

Les degrés de liberté représentatifs de la position et de l'orientation du stent comprennent au moins une position 3D d'au moins un point caractéristique du stent, et une rotation propre r_{x'} du stent autour de son axe central. Selon différents modes de réalisation, la position 6D du stent peut être définie de différentes manières. Par exemple, elle peut être définie par la position 3D d'au moins deux points caractéristiques du stent et un angle de rotation propre, ou par la position 3D d'un seul point caractéristique du stent, et 3 angles de rotation. L'homme de l'art pourra sans effort définir le type de modélisation des stents correspondant à ses besoins.

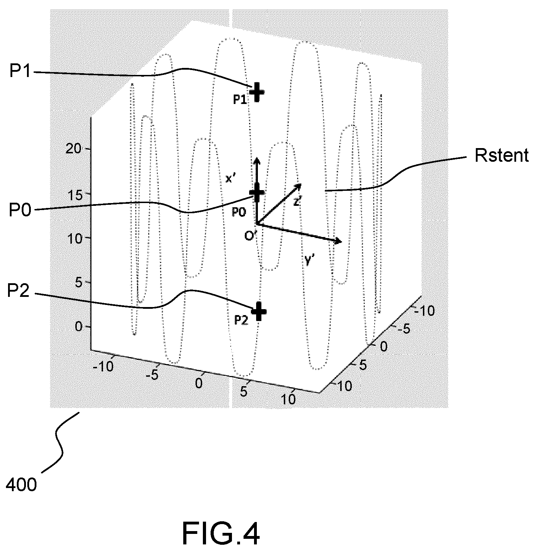

La figure 4 représente une modélisation d'un stent au sein d'un modèle d'endoprothèse selon un ensemble de modes de mise en œuvre de l'invention.

La figure 4 représente la modélisation 400 d'un stent au sein d'un modèle d'endoprothèse 331.

Dans cet exemple, le stent est associé à un référentiel propre Rₛₜₑₙₜ comprenant 3 axes (x', y', z') initialement alignés avec les axes (x, y, z) du référentiel Rᵢₘ. La rotation propre du stent r_{x'} est définie autour de l'axe central (que l'on peut également désigner axe vertical) x'. Ce modèle simplifié comprend la position 3D de trois points caractéristiques P0, P1, P2, correspondant respectivement au centre, à l'extrémité supérieure et à l'extrémité inférieure du stent le long de son axe central x'. Dans cet exemple, la position et l'orientation du stent sont donc définies par 10 degrés de liberté (9 degrés pour les 3 positions 3D, et un degré pour la rotation).

Cette utilisation de 3 points caractéristiques permet d'améliorer encore la détermination de la position et de l'orientation du stent.

Cependant, cette modélisation des position et orientation d'un stent est donnée à titre d'exemple non limitatif uniquement, et les position et orientation d'un stent pourraient être représentées d'autres manières. Par exemple, elles pourraient être représentées par la position du point caractéristique P0 et 3 rotations, ou les positions des points caractéristiques P1 et P2 et la rotation propre autour de l'axe x'.

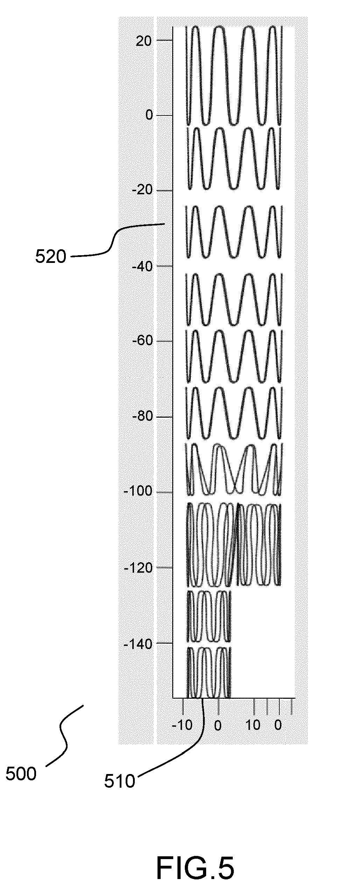

La figure 5 représente le déploiement d'un stent dans le cadre d'un modèle de l'endoprothèse.

Dans un ensemble de modes de réalisation de l'invention, le déploiement du stent est défini par une valeur unique. Cette valeur peut être par exemple le diamètre du stent, un pourcentage ou un ratio de déploiement.

Le diagramme 500 représente le déploiement simplifié de plusieurs stents. L'axe 510 représente le déploiement du stent, en mm L'axe 520 représente la position et la longueur de chaque stent.

Il est également possible, selon différents modes de réalisation de l'invention, de représenter le déploiement du stent selon un nombre limité de valeurs représentant le déploiement du stent en différents points caractéristiques autour de son axe central. Par exemple, le déploiement du stent peut être représenté par une valeur de déploiement à l'extrémité supérieure du stent, et une valeur de déploiement à son extrémité inférieure.

La représentation du déploiement du stent par au moins une valeur représentative d'un niveau de déploiement du stent autour de son axe central permet donc, en combinaison avec les valeurs de position et d'orientation, de représenter le déploiement de chaque stent au sein de l'endoprothèse de manière très synthétique, à l'aide d'un nombre limité de paramètres, dont les valeurs peuvent être extraites d'une image péri-opératoire en 2D, pour la plupart d'entre eux.

Le modèle 331 de l'endoprothèse permet donc une modélisation simplifiée de l'endoprothèse, permettant de représenter globalement le positionnement de l'endoprothèse, avec un nombre de degrés de liberté limité.

Revenant à la figure 3, la méthode 300 comprend une étape 330 de détermination, à partir de l'image 2D et pour chaque stent, de l'au moins une position de l'au moins un point caractéristique selon les axe vertical (x) et horizontal (y) et l'au moins une valeur de déploiement dudit stent.

Cette étape consiste à identifier toutes les valeurs du modèle d'endoprothèse pouvant être directement obtenues à partir de l'image 2D.

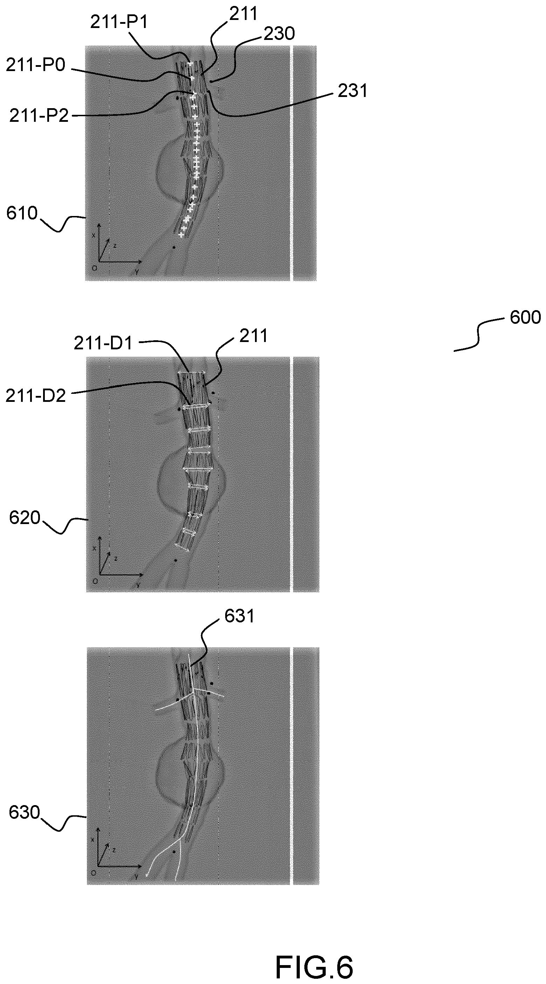

La figure 6 représente la détermination, à partir de l'image 2D, des positions verticales et horizontales des points caractéristiques des stents, ainsi que du déploiement de ceux-ci.

Plus précisément, la figure 6 représente l'image de la figure 2c, sur laquelle sont relevées différents paramètres permettant de modéliser le déploiement de l'endoprothèse en 3D. La figure 6 montre ainsi la détection de certaines valeurs sur l'image péri-opératoire de l'aorte, sur une image 2D semblable à celles que pourrait visualiser un chirurgien en temps réel. Bien que la figure 2c et la figure 6 montrent une endoprothèse déployée dans une maquette de l'aorte, les éléments présentés en figure 6 sont également applicable à une image montrant en superposition une l'aorte révélée grâce au produit de contraste, en superposition avec une image de l'endoprothèse, ainsi qu'à des images de l'endoprothèse non ou partiellement déployée.

Les 3 vignettes 610, 620 et 630 représentent respectivement 3 exemples de détermination, sur l'image 200, des positions des points caractéristiques des stent, déploiement des stents et l'association avec la ligne centrale de l'aorte.

En particulier, l'image 2D permet d'obtenir directement, comme représenté à la figure 2b, les positions de chacun des points caractéristiques des stents selon les axes x et y du référentiel Rᵢₘ. Le relevé peut se faire manuellement à partir des informations représentées sur l'écran, ou de manière semi-automatique à partir d'un traitement en détectant la position des centres de gravité des stents. Dans l'exemple représenté en figure 6, le stent 211 est associé à 3 points caractéristiques 211-P1, 211-P0 et 211-P2, selon le modèle représenté en figure 4. L'analyse de l'image 200b permet ainsi de déduire directement les positions des points caractéristiques 211-P0, 211-P1 et 211-P2 selon les axes x et y, pour chaque stent. Par exemple, ces points peuvent correspondre aux points 211b-0, 211b-1 et 211b-2 de l'image 200b, dont la position peut être déterminée directement par analyse de l'image 200b. Selon différents modes de réalisation de l'invention, les positions x,y des points caractéristiques peuvent être déterminées par rapport à une origine unique du référentiel Rᵢₘ, ou par rapport à un point central de l'aorte. Les stents sont alors positionnés dans la simulation grâce aux positions des points caractéristiques ainsi déterminées.

La vignette 620 représente la détermination, pour chaque stent, d'au moins une valeur de déploiement dudit stent autour de son axe central. Dans l'exemple de la figure 6, chaque stent est associé à une valeur de déploiement de son extrémité supérieure, et une valeur de déploiement de son extrémité inférieure. Par exemple, le stent 211 est associé à une valeur de déploiement 211-D1 à son extrémité supérieure, et une valeur de déploiement 211-D2 à son extrémité inférieure. Ici encore, les valeurs de déploiement du stent peuvent être obtenues de manière directe par analyse de l'image 200. Dans l'exemple de la vignette 620, les stents sont déployés, et la valeur de déploiement peut être obtenue directement en mesurant le déploiement des stents sur l'image, manuellement ou automatiquement par analyse d'image.

Dans des modes de réalisation dans lesquels l'endoprothèse n'est pas ou pas totalement déployée, la valeur de déploiement peut être déterminée comme le diamètre de l'aorte au point de déploiement, si le diamètre maximal de déploiement de l'endoprothèse est supérieur ou égale audit diamètre de l'aorte au point de déploiement. Dans le cas contraire, la valeur de déploiement correspond au déploiement maximal de l'endoprothèse. En effet, le diamètre de déploiement final de l'endoprothèse en ce point sera, dans ce cas, contraint soit par le diamètre de l'aorte, soit par celui du stent.

La vignette 630 représente l'association entre les points caractéristiques des stents et la ligne centrale de l'aorte.

La ligne centrale de l'aorte peut être obtenue par projection d'une ligne centrale 3D préalablement obtenue, ou par une méthode de squelettisation de l'image 2D (de nombreuses méthodes de squelettisation de l'image 2D sont connues, comme par exemple celle décrite par Couprie, M., Coeurjolly, D., & Zrour, R. (2007). Discrete bisector function and Euclidean skeleton in 2D and 3D. Image and Vision Computing, 25(10), 1543-1556.) Dans un ensemble de modes de réalisation de l'invention la position des points caractéristiques du stent selon l'axe z est initialisée sur la ligne centrale de l'aorte.

Comme montré à la figure 6, l'étape 330 de détermination des positions horizontale, verticale et des déploiements des stents peut se faire par analyse directe d'une image péri-opératoire en 2D, soit manuellement, soit de manière automatique par analyse de l'image.

Cette détermination est donc effectuée de manière très rapide. Ainsi, l'étape 330 peut s'effectuer en temps réel dès la prise de vue par capteur de l'image 2D. De plus, cette étape permet une grande précision, inférieure au millimètre, sur les valeurs déterminées.

Certaines caractéristiques du modèle de l'endoprothèse ne peuvent pas être déterminées directement par l'analyse directe de l'image. Par exemple, la position des points caractéristiques sur l'axe de profondeur z, et la rotation propre des stents ne peuvent pas être déterminés directement. Ces valeurs peuvent être initialisées à des valeurs par défaut. Par exemple, les positions des points sur l'axe de profondeur peuvent être initialisés pour que les points soient situés sur la ligne centrale de l'aorte, et la rotation propre du stent autour de l'axe x' peut être initialisée à 0.

Dans un ensemble de modes de réalisation de l'invention, l'ensemble de l'endoprothèse est modélisée sous la forme d'un modèle éléments finis dans lequel chaque stent est modélisé par un élément poutre, et les stents sont liés entre eux par de petits éléments poutres.

Dans un ensemble de modes de réalisation de l'invention, une pluralité d'éléments poutres de liaison (par exemple 5) sont disposés en enfilade entre les extrémités des éléments poutres représentant les stents. Ces éléments poutres de liaison peuvent être associés à des matrices de raideur dont les caractéristiques mécaniques sont différentes de celles des matrices de raideurs des éléments poutres représentant les stent. Ainsi, ces éléments poutres de liaison auront un comportement moins rigide. Ceci permet de modéliser de manière précise l'équilibre mécanique entre les stents successifs.

Les stents dont le diamètre maximal de déploiement est supérieur ou égal au diamètre de l'aorte, à l'endroit de déploiement, sont considérés comme contraints : la position de leurs points caractéristiques est fixée selon les axes x, y et z. En effet, leur position sera complètement contrainte par le volume de l'aorte lors de leur déploiement. En revanche, ceux dont le diamètre maximal de déploiement est inférieur au diamètre de l'aorte sont considérés comme « libres » : leurs points caractéristiques sont contraints en déplacement selon les axe horizontal x et vertical y, mais libres de déplacement selon l'axe z. Leur position selon l'axe de profondeur z est alors définie par l'équilibre mécanique du modèle élément fini.

Ceci permet d'obtenir une position précise en 3D des stents dont la position n'est pas complètement contrainte par le volume de l'aorte, tout en prenant en compte l'équilibre des forces mécaniques au sein de l'aorte.

Le positionnement des points caractéristiques des stents sur la ligne centrale de l'aorte fournit un bon compromis entre temps de calcul et précision de la méthode.

Revenant à la figure 3, la méthode 300 comprend une étape 340 de détermination de l'angle de rotation propre, d'au moins un stent de l'endoprothèse.

Selon différents modes de réalisation de l'invention, cette étape, exécutée sur chaque stent séparément, peut être exécutée sur tout ou partie des stents de l'endoprothèse. Par exemple, elle peut être exécutée sur chaque stent de l'endoprothèse. Elle peut aussi être exécutée sur certains stents critiques seulement, par exemple des stents comprenant une fenêtre à positionner face à un ostia. Le chirurgien peut également sélectionner manuellement les stents sur lesquels exécuter cette étape selon ses besoins.

Dans un ensemble de modes de réalisation de l'invention, la détermination, pour au moins un stent, de son angle de rotation propre r_{x'} comprend la détermination de l'angle de rotation propre r_{x'} pour lequel la projection de la position d'un au moins marqueur radio-opaque correspond le plus à l'image de ce marqueur sur l'image 2D. Par exemple, dans l'image 200 également représentée en vignettes 610, 620 et 630 de [Fig.6], le stent 211 comprend un certain nombre de marqueurs radio-opaques, tels que les marqueurs 230 et 231. La position de ces marqueurs radio-opaques et connue dans le modèle simplifiée du stent. Ainsi, connaissant les valeurs de déploiement du stent, il est possible, pour chaque valeur de l'angle de rotation propre r_{x'}, de projeter la position 3D du marqueur radio-opaque sur l'image 2D, et de vérifier si elle concorde avec la position du marqueur radio-opaque sur l'imagerie 2D. Cette comparaison peut être effectuée pour un ou plusieurs marqueurs.

La détermination de r_{x'} peut ainsi se faire de différentes manières. Par exemple, l'angle de rotation propre r_{x'} peut être obtenu par l'exécution d'une boucle de minimisation de la distance entre les projections des positions 3D et les images des au moins un marqueur sur l'image 2D, en fonction de I 'angle de rotation propre r_{x'}.

Ainsi, l'angle r_{x'} peut être modifié de manière itérative, et à chaque itération la différence de position, pour chaque marqueur, entre la projection de sa position 3D dans le modèle sur l'image 2D, et son image prise par rayon X peut être calculée. Si plusieurs marqueurs sont utilisés, les valeurs absolues des différences de positions peuvent être additionnées ou prises séparément. Ainsi, les valeurs des angles r_{x'} peuvent être déterminées de manières itérative pour minimiser les différences de positions entre les projections des positions 3D et les images du ou des marqueurs sur l'imagerie 2D, par exemple par le biais d'un algorithme du type descente de gradient, de méthode stochastiques; ou tout autre algorithme permettant d'identifier un minimum global des différences entre les projections des positions 3D des marqueurs et leurs images, en fonction de la rotation propre r_{x'}. Selon différents modes de réalisation de l'invention, de tels algorithmes peuvent être initialisés avec un unique point de départ, ou avec de multiples points de départ afin d'éviter de converger vers un minimum local. Tout algorithme de minimisation peut être utilisé ici.

Ceci fournit une manière simple et rapide de déterminer la rotation propre r_{x'} d'un stent.

La méthode 300 comprend ensuite la simulation du déploiement dans le modèle 3D de l'aorte 311, pour chaque stent, d'un modèle du stent 351 formé d'une pluralité d'éléments poutres, et initialisé à partir du modèle 331 de l'endoprothèse 210.

Cette étape consiste, une fois déterminées les valeurs des paramètres du modèle 331 de l'endoprothèse, dans lequel chaque stent est représenté de manière simplifiée, d'utiliser ces valeurs de paramètres pour initialiser un modèle plus complexe de chaque stent, et de simuler son déploiement dans le modèle 3D de l'aorte.

Dans les cas où l'endoprothèse est déjà déployée, ceci permet de modéliser en 3D le déploiement actuel de l'endoprothèse. Dans les cas où l'endoprothèse n'est pas ou pas complètement déployée, ceci permet au chirurgien de visualiser ce que sera le déploiement de l'endoprothèse s'il décide de la déployer dans sa position courante.

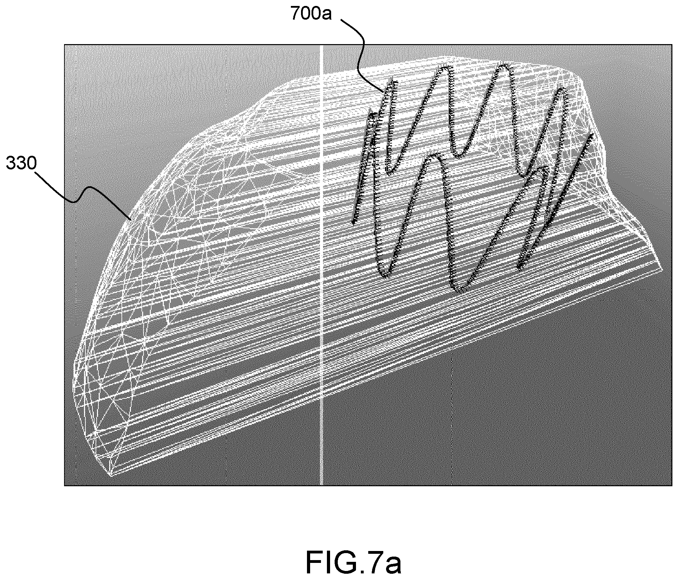

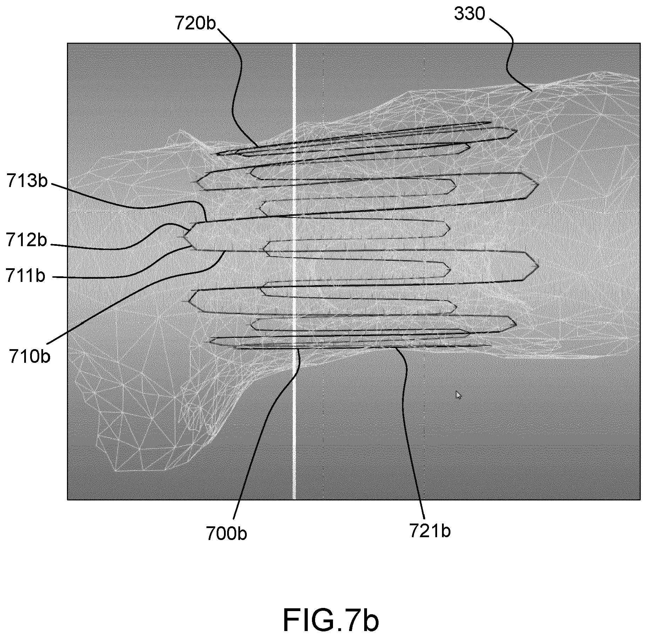

Les figures 7a et 7b représentent deux exemples de modélisation du déploiement d'un stent selon un ensemble de modes de mise en œuvre de l'invention.

Le modèle 351 d'un stent est formé d'une pluralité d'éléments poutres modélisant les stents. Le nombre d'éléments poutres peut être variable en fonction de la complexité voulue. Un modèle 351 d'un stent peut par exemple être formé d'une centaine s'éléments poutres.

La figure 7a représente l'initialisation d'un modèle 700a (correspondant à une instance du modèle 351) dans le modèle 3D de l'aorte 330. Comme expliqué ci-dessus, les étapes 330 et 340 permettent de déterminer pour chaque stent, des caractéristiques d'un modèle simplifié : position des points caractéristiques, rotation autour de l'axe central... Ces valeurs permettent d'initialiser la position et l'orientation des éléments poutres du modèle 351, 700a représentant la structure du stent.

Dans l'exemple de la figure 7a, le stent n'est pas encore déployé. Il suffit alors, pour simuler le déploiement du stent, de simuler l'extension des éléments poutres de la position d'initialisation jusqu'aux limites du modèle 3D 330 de l'aorte.

La figure 7b représente un modèle de stent déployé dans le modèle 3D de l'aorte 330. Comme expliqué ci-dessus, le modèle 700b est formé d'une pluralité d'éléments poutres, par exemple les éléments 710b, 711b, 712b, 713b. Le stent peut alors être déployé, soit jusqu'à extension maximale, soit jusqu'à ce que les éléments poutres atteignent les limites de l'aorte, par exemple aux points 720b, 721b.

Le modèle éléments finis permet de prendre en compte en même temps les contraintes au sein du stent, et de l'interaction entre le stent et l'aorte.

Le modèle éléments finis représentant la structure du stent étant initialisé à partir du modèle simplifié préalablement calculé, la simple simulation du déploiement du stent est rapide à mettre en œuvre, tout en modélisant de manière très précise le déploiement du stent.

La méthode 300, dans son ensemble, permet donc un calcul à la fois fiable et rapide du déploiement d'une endoprothèse dans l'aorte. Par exemple, la méthode peut s'exécuter en une trentaine de secondes sur des moyens de calcul conventionnels, ce qui permet une modélisation du déploiement de l'endoprothèse quasiment en temps réel.

Ce résultat est permis par le fait que le modèle complet modélisant la structure de chaque stent (modèle 351, 700a, 700b), qui comprend de nombreux éléments, est initialisé par les étapes précédentes et n'a besoin que d'être déployé. S'il est possible de modéliser directement le déploiement d'un modèle complet représentant la structure de chaque stent de l'endoprothèse sans hypothèse préalable, ceci prendrait un temps considérable incompatible avec l'exécution d'une opération en temps réel.

Revenant à la figure 3, la méthode 300 comprend une étape d'affichage 360 du modèle déployé des stents. Cet affichage peut se faire, soit en 2D en superposant la projection du déploiement des stents sur une image péri-opératoire en 2D telle que l'image 200, ou en 3D en affichant le déploiement des stents en 3D et le modèle de l'aorte 330. L'affichage peut par exemple s'effectuer sur les écrans 121 d'un dispositif d'imagerie médicale.

Etant donné que la méthode permet de simuler le déploiement de l'endoprothèse de manière très précise et en temps réel, l'affichage 360 permet au chirurgien de disposer en temps réel d'une visualisation du déploiement de l'endoprothèse à partir de sa position actuelle. Ceci permet ainsi de considérablement améliorer la précision du traitement effectué par le chirurgien.

Dans le cas où la méthode est appliquée à une endoprothèse déployée, le chirurgien peut visualiser le déploiement en 3D. Dans le cas où la méthode est appliquée à une endoprothèse non déployée, le chirurgien peut visualiser ce que sera le déploiement de l'endoprothèse, en 3D, s'il décide de la déployer à partir de la position courante.

De plus, cette méthode requiert une unique prise de vue pour simuler le déploiement d'une endoprothèse, ce qui évite d'utiliser des injections multiples de produit de contraste.

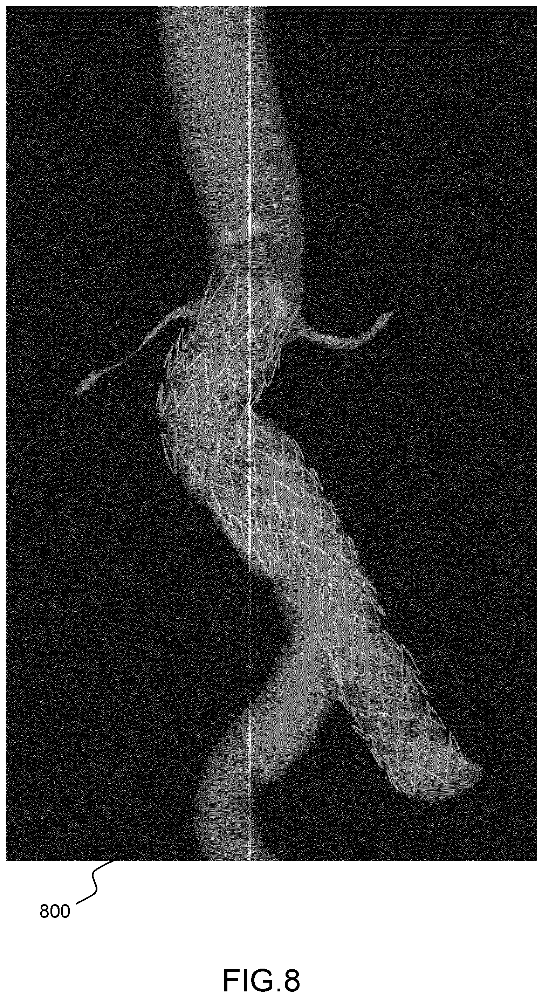

La figure 8 représente une superposition de la simulation de déploiement d'un stent sur une image péri-opératoire selon un ensemble de modes de mise en œuvre de l'invention.

L'image 800 représente la superposition de la simulation du déploiement de l'endoprothèse sur l'imagerie 2D issue d'un C-arm. Comme expliqué précédemment, l'invention permet également de représenter en 3D la simulation du déploiement de l'endoprothèse sur le modèle 3D de l'aorte. Dans le cas d'une visualisation 3D, le chirurgien peut manipuler la représentation pour visualiser la prédiction du déploiement selon différents angles de vue.

Dans les deux cas, ceci permet au chirurgien de visualiser la simulation du déploiement de l'endoprothèse en temps réel.

Les exemples ci-dessus démontrent la capacité de l'invention à permettre, de déterminer le déploiement d'une endoprothèse. Ils ne sont cependant donnés qu'à titre d'exemple et ne limitent en aucun cas la portée de l'invention, définie dans les revendications ci-dessous.

## Revendications

1. Méthode mise en œuvre par ordinateur (300) comprenant :
- la capture (310), par un dispositif d'imagerie par rayons X, d'une image 2D (311) d'une structure vasculaire, et d'une endoprothèse comprenant une pluralité de stents dans la structure vasculaire, par rayon X ;
- l'obtention d'un modèle numérique 3D de la structure vasculaire (321) ;
- l'obtention d'un modèle numérique (331) de l'endoprothèse (210) dans la structure vasculaire;
- la détermination (330, 340), à partir de l'image 2D et pour chaque stent, de caractéristiques du modèle numérique (331) de l'endoprothèse comprenant au moins une position, un angle de rotation propre (r_{x'}) autour de son axe central (x')., et au moins une valeur de déploiement ;
- la simulation du déploiement (350) dans le modèle numérique 3D de la structure vasculaire (311), pour chaque stent, d'un modèle numérique du stent (351) représentant la structure du stent, ledit modèle numérique du stent étant initialisé à partir du modèle numérique (331) de l'endoprothèse (210) ;
- l'affichage (360) des modèles numériques de stents déployés dans l'étape de simulation ;
ladite méthode étant **caractérisée en ce que** la détermination (340), pour au moins un stent, de son angle de rotation propre (r_{x'}) comprend la détermination de l'angle de rotation propre (r_{x'}) pour lequel au moins une projection de position 3D d'au moins deux marqueurs radio-opaques, sur le stent correspond le plus à au moins une image desdits marqueurs sur l'image 2D.

2. Méthode selon la revendication 1, dans laquelle :
- l'image 2D (311) définit un référentiel 3D (Rᵢₘ) comprenant un axe vertical (x) et un axe horizontal (y) et un axe de profondeur (z) de la prise de vue ;
- dans le modèle numérique de l'endoprothèse, chaque stent est défini par :
- au moins une position d'au moins un point caractéristique (P0, P1, P2) et une orientation définies par au moins 6 degrés de liberté dont :
- la position 3D d'un premier point caractéristique (P0) du stent dans le référentiel ;
- l'angle de rotation propre (r_{x'}) ;
- l'au moins une valeur de déploiement du stent, définie autour de son axe central ;
- le modèle numérique du stent est formé d'une pluralité d'éléments poutres liés entre eux ;
ladite méthode comprenant :
- la détermination (330, 340), à partir de l'image 2D et pour chaque stent, d'au moins une position, au moins une orientation, et au moins une valeur de déploiement ; de l'au moins un point caractéristique selon les axe vertical (x) et horizontal (y) et l'au moins une valeur de déploiement dudit stent ;
- la détermination (340), pour au moins un stent, de son angle de rotation propre (rₓ').

3. Méthode selon l'une des revendications 1 ou 2 dans laquelle la position et l'orientation de chaque stent sont définies par les positions 3D de trois points caractéristiques (P0, P1, P2) correspondant respectivement au centre, à l'extrémité supérieure et à l'extrémité inférieure du stent le long de son axe central (x'), et la rotation propre du stent

4. Méthode selon l'une des revendications 1 à 3, dans laquelle l'angle de rotation propre (r_{x'}) est obtenu par l'exécution d'une boucle de minimisation de la distance entre l'au moins une projection et l'au moins une image de l'au moins un marqueur sur l'image 2D, en fonction de l'angle de rotation propre (r_{x'}).

5. Méthode selon l'une des revendications 1 à 4, dans laquelle la détermination (340), pour au moins un stent, de son angle de rotation propre (r_{x'}) comprend la modélisation du stent sous la forme d'un élément poutre d'un modèle éléments finis, entre un point caractéristique représentant l'extrémité supérieure de l'axe central du stent (P1) et un point caractéristique représentant l'extrémité inférieure de l'axe central du stent (P2), dans lequel les points caractéristiques sont libres de déplacements selon l'axe de profondeur (z).

6. Méthode selon la revendication 5, dans laquelle :
- l'endoprothèse est représentée sous la forme d'un modèle éléments finis dans lequel :
- chaque stent est modélisé par un élément poutre, les stents successifs étant liés entre eux par au moins un élément poutre ;
- les positions des points caractéristiques des stents dont le diamètre maximal de déploiement est supérieur ou égal au diamètre de la structure vasculaire à la position de déploiement sont fixés selon les axes vertical (x), horizontal (y) et de profondeur (z) ;
- les positions des points caractéristiques des stents dont le diamètre maximal de déploiement est inférieur au diamètre de la structure vasculaire à la position de déploiement sont fixés selon les axes vertical (x), horizontal (y) et libres en déplacement selon l'axe de profondeur (z) ;
- les position des points caractéristiques des stents dont le diamètre maximal de déploiement est inférieur au diamètre de la structure vasculaire à la position de déploiement selon l'axe de profondeur (z) sont déterminés par l'équilibre mécanique du modèle éléments finis.

7. Méthode selon l'une des revendications 1 à 6, dans laquelle l'affichage (360) des modèles numériques de stents déployés comprend l'affichage en superposition de la projection des modèles numériques des stents (351) sur l'image 2D (311) de la structure vasculaire.

8. Méthode selon l'une des revendications 1 à 6, dans laquelle l'affichage (360) des modèles numériques de stents déployés comprend l'affichage en 3D des modèles numériques de stents déployés et du modèle numérique 3D de la structure vasculaire (321).

9. Méthode selon l'une des revendications 1 à 8, dans laquelle le modèle numérique 3D de la structure vasculaire (321) est un modèle éléments finis représentant la ligne centrale de la structure vasculaire par des éléments poutres.

10. Produit programme d'ordinateur comprenant des éléments de code informatique configurés pour exécuter une méthode selon l'une des revendications 1 à 9 lorsque ledit programme est exécuté sur une unité de calcul d'un dispositif de calcul.

11. Dispositif (120) comprenant :
- au moins un port d'entrée configuré pour recevoir d'une image 2D (311) de la structure vasculaire capturée par rayon X ;
- au moins une unité de calcul configurée pour exécuter une méthode selon l'une des revendications 1 à 9.

## Patentansprüche

1. Verfahren, das von einem Computer (300) umgesetzt wird, umfassend:
- Erfassen (310), mit einer Röntgenbildgebungsvorrichtung, von einem 2D-Bild (311) von einer Gefäßstruktur und von einer Endoprothese, die eine Vielzahl von Stents in der Gefäßstruktur umfasst, mittels Röntgenstrahlen;
- Erhalten eines digitalen 3D-Modells der Gefäßstruktur (321);
- Erhalten eines digitalen Modells (331) der Endoprothese (210) in der Gefäßstruktur;
- Bestimmen (330, 340), anhand des 2D-Bilds und für jeden Stent, von Eigenschaften des digitalen Modells (331) der Endoprothese, die mindestens eine Position, einen Eigendrehwinkel (r_{x'}) um seine Mittelachse (x') und mindestens einen Entfaltungswert umfassen;
- Simulieren des Entfaltens (350) in dem digitalen 3D-Modell der Gefäßstruktur (311) für jeden Stent von einem digitalen Modell des Stents (351), das die Struktur des Stents darstellt, wobei das digitale Modell des Stents anhand des digitalen Modells (331) der Endoprothese (210) initialisiert wird;
- Anzeigen (360) der digitalen Modelle von entfalteten Stents im Simulationsschritt;
wobei das Verfahren **dadurch gekennzeichnet ist, dass** das Bestimmen (340), für mindestens einen Stent, seines Eigendrehwinkels (r_{x'}) das Bestimmen des Eigendrehwinkels (r_{x'}) umfasst, bei dem mindestens eine 3D-Positionsprojektion von mindestens zwei röntgendichten Markern auf den Stent am ehesten mindestens einem Bild der Marker in dem 2D-Bild entspricht.

2. Verfahren nach Anspruch 1, wobei:
- das 2D-Bild (311) ein 3D-Bezugsrahmensystem (Rᵢₘ) definiert, das eine Vertikalachse (x) und eine Horizontalachse (y) und eine Tiefenachse (z) der Aufnahme umfasst;
- in dem digitalen Modell der Endoprothese jeder Stent definiert ist durch:
- mindestens eine Position von mindestens einem charakteristischen Punkt (P0, P1, P2) und eine Ausrichtung, die durch mindestens 6 Freiheitsgrade definiert sind, darunter:
- die 3D-Position eines ersten charakteristischen Punkts (P0) des Stents im Bezugssystem;
- der Eigendrehwinkel (r_{x'});
- mindestens einen Entfaltungswert des Stents, der um seine Mittelachse herum definiert ist;
- das digitale Modell des Stents aus einer Vielzahl von Balkenelementen aufgebaut ist, die miteinander verbunden sind;
wobei das Verfahren Folgendes umfasst:
- Bestimmen (330, 340), anhand des 2D-Bilds und für jeden Stent, von mindestens einer Position, mindestens einer Ausrichtung und mindestens einem Entfaltungswert, von dem mindestens einen charakteristischen Punkt entlang der Vertikalachse (x) und der Horizontalachse (y) und von dem mindestens einem Entfaltungswert des Stents;
- Bestimmen (340), für mindestens einen Stent, von seinem Eigendrehwinkel (r_{x'}).

3. Verfahren nach Anspruch 1 oder 2, wobei die Position und die Ausrichtung von jedem Stent durch die 3D-Position von drei charakteristischen Punkten (P0, P1, P2), die der Mitte, dem oberen Ende beziehungsweise dem unteren Ende des Stents entlang seiner Mittelachse (x') entsprechen, sowie die Eigendrehung des Stents definiert sind.

4. Verfahren nach einem der Ansprüche 1 bis 3, wobei der Eigendrehwinkel (r_{x'}) durch Ausführen einer Schleife zur Minimierung des Abstands zwischen der mindestens einen Projektion und dem mindestens einen Bild von dem mindestens einen Marker im 2D-Bild in Abhängigkeit vom Eigendrehwinkel (r_{x'}) erhalten wird.

5. Verfahren nach einem der Ansprüche 1 bis 4, wobei das Bestimmen (340), für mindestens einen Stent, seines Eigendrehwinkels (r_{x'}) das Modellieren des Stents in Form eines Balkenelements eines Finite-Elemente-Modells zwischen einem charakteristischen Punkt, der das obere Ende der Mittelachse des Stents (P1) darstellt, und einem charakteristischen Punkt, der das untere Ende der Mittelachse des Stents (P2) darstellt, umfasst, wobei die charakteristischen Punkte entlang der Tiefenachse (z) frei beweglich sind.

6. Verfahren nach Anspruch 5, wobei:
- die Endoprothese in Form eines Finite-Elemente-Modells dargestellt ist, wobei:
- jeder Stent modellhaft durch ein Balkenelement abgebildet ist, wobei die aufeinanderfolgenden Stents über mindestens ein Balkenelement miteinander verbunden sind;
- die Position der charakteristischen Punkte der Stents, deren größter Durchmesser bei der Entfaltung größer oder gleich dem Durchmesser der Gefäßstruktur an der Entfaltungsposition ist, auf der Vertikalachse (x), Horizontalachse (y) und Tiefenachse (z) fest ist;
- die Position der charakteristischen Punkte der Stents, deren größter Durchmesser bei der Entfaltung kleiner als der Durchmesser der Gefäßstruktur an der Entfaltungsposition ist, auf der Vertikalachse (x), der Horizontalachse (y) fest ist, und frei beweglich nach der Tiefenachse (z) ist;
- die Position der charakteristischen Punkte der Stents, deren größter Durchmesser bei der Entfaltung kleiner als der Durchmesser der Gefäßstruktur an der Entfaltungsposition ist, auf der Tiefenachse (z) durch das mechanische Gleichgewicht des Finite-Elemente-Modells bestimmt wird.

7. Verfahren nach einem der Ansprüche 1 bis 6, wobei das Anzeigen (360) der digitalen Modelle von entfalteten Stents das Anzeigen der Projektion der digitalen Modelle der Stents (351) als Überlagerung über das 2D-Bild (311) der Gefäßstruktur umfasst.

8. Verfahren nach einem der Ansprüche 1 bis 6, wobei das Anzeigen (360) der digitalen Modelle von entfalteten Stents das dreidimensionale Anzeigen der digitalen Modelle von entfalteten Stents und des digitalen 3D-Modells der Gefäßstruktur (321) umfasst.

9. Verfahren nach einem der Ansprüche 1 bis 8, wobei das digitale 3D-Modell der Gefäßstruktur (321) ein Finite-Elemente-Modell ist, das die Mittellinie der Gefäßstruktur mit Balkenelementen darstellt.

10. Computerprogrammprodukt, das Computercodeelemente umfasst, die so ausgelegt sind, dass sie ein Verfahren nach einem der Ansprüche 1 bis 9 ausführen, wenn das Programm in einer Recheneinheit einer Rechenvorrichtung ausgeführt wird.

11. Vorrichtung (120), umfassend:
- mindestens einen Eingangsanschluss, der so ausgelegt ist, dass er ein 2D-Bild (311) der Gefäßstruktur empfängt, das mittels Röntgenstrahlen aufgenommen wird;
- mindestens eine Berechnungseinheit, die so ausgelegt ist, dass sie ein Verfahren nach einem der Ansprüche 1 bis 9 ausführt.

## Claims

1. Method implemented by computer (300) comprising:
- the capture (310), by an X-ray imaging device, of a 2D image (311) of a vascular structure, and an endoprosthesis comprising a plurality of stents in the vascular structure, by X-ray;
- the obtaining of a 3D digital model of the vascular structure (321);
- the obtaining of a digital model (331) of the endoprosthesis (210) in the vascular structure;
- the determination (330, 340), from the 2D image and for each stent, of features of the digital model (331) of the endoprosthesis comprising at least one position, a specific rotation angle (r_{x'}) about its central axis (x'), and at least one deployment value;
- the simulation of the deployment (350) in the 3D digital model of the vascular structure (311), for each stent, of a digital model of the stent (351) representing the structure of the stent, said digital model of the stent being initialized from the digital model (331) of the endoprosthesis (210);
- the displaying (360) of digital models of stents deployed in the simulation step;
said method being **characterized in that** the determination (340), for at least one stent, of its specific rotation angle (r_{x'}) comprises the determination of the specific rotation angle (r_{x'}) for which at least one 3D position projection of at least two radiopaque markers, on the stent, corresponds the most to at least one image of said markers on the 2D image.

2. Method according to claim 1, wherein:
- the 2D image (311) defines a 3D reference point (Rᵢₘ) comprising a vertical axis (x) and a horizontal axis (y) and a depth axis (z) of the image acquisition;
- in the digital model of the endoprosthesis, each stent is defined by:
- at least one position of at least one characteristic point (P0, P1, P2) and an orientation defined by at least 6 degrees of freedom, of which:
- the 3D position of a first characteristic point (P0) of the stent in the reference point;
- the specific rotation angle (r_{x'});
- the at least one deployment value of the stent, defined about its central axis;
- the digital model of the stent is formed from a plurality of beam elements connected to one another;
said method comprising:
- the determination (330, 340), from the 2D image and for each stent, of at least one position, at least one orientation, and at least one deployment value; of the at least one characteristic point along the vertical (x) and horizontal (y) axis and the at least one deployment value of said stent;
- the determination (340), for at least one stent, of its specific rotation angle (r_{x'}).

3. Method according to any one of claims 1 or 2, wherein the position and the orientation of each stent are defined by the 3D positions of three characteristic points (P0, P1, P2) corresponding respectively to the center, at the upper end and at the lower end of the stent along its central axis (x'), and the specific rotation of the stent.

4. Method according to any one of claims 1 to 3, wherein the specific rotation angle (r_{x'}) is obtained by the execution of a loop to minimize the distance between the at least one projection and the at least one image of the at least one marker on the 2D image, as a function of the specific rotation angle (r_{x'}).

5. Method according to any one of claims 1 to 4, wherein the determination (340), for at least one stent, of its specific rotation angle (r_{x'}) comprises the modeling of the stent in the form of a beam element of a finite element model, between a characteristic point representing the upper end of the central axis of the stent (P1) and a characteristic point representing the lower end of the central axis of the stent (P2), wherein the characteristic points are free of displacements along the depth axis (z).

6. Method according to claim 5, wherein:
- the endoprosthesis is represented in the form of a finite element model, wherein:
- each stent is modeled by a beam element, the successive stents being connected to one another by at least one beam element;
- the positions of the characteristic points of the stents, the maximum deployment diameter of which is greater than or equal to the diameter of the vascular structure at the deployment position are fixed along the vertical (x), horizontal (y) and depth (z) axes;
- the positions of the characteristic points of the stents, the maximum deployment diameter of which is less than the diameter of the vascular structure at the deployment position are fixed along the vertical (x), horizontal (y) axes and free in displacement along the depth (z) axis;
- the positions of the characteristic points of the stents, the maximum deployment diameter of which less than the diameter of the vascular structure at the deployment position along the depth (z) axis are determined by the mechanical balance of the finite element model.

7. Method according to any one of claims 1 to 6, wherein the displaying (360) of digital models of deployed stents comprises the superimposed displaying of the projection of the digital models of the stents (351) on the 2D image (311) of the vascular structure.

8. Method according to any one of claims 1 to 6, wherein the displaying (360) of digital models of deployed stents comprises the 3D displaying of digital models of deployed stents and of the 3D digital model of the vascular structure (321).

9. Method according to any one of claims 1 to 8, wherein the 3D digital model of the vascular structure (321) is a finite element model representing the central line of the vascular structure by beam elements.

10. Computer program product comprising computerized code elements configured to execute a method according to any one of claims 1 to 9, when said program is executed on a calculation unit of a calculation device.

11. Device (120) comprising:
- at least one input port configured to receive a 2D image (311) of the vascular structure captured by X-ray;
- at least one calculation unit configured to execute a method according to any one of claims 1 to 9.
